(19) Europäisches Patentamt
European Patent Office
Office européen des brevets

(11) Veröffentlichungsnummer: **0 116 167**
**B1**

(12) # EUROPÄISCHE PATENTSCHRIFT

(45) Veröffentlichungstag der Patentschrift: **26.02.86**

(51) Int. Cl.⁴: **C 07 C 149/24,** C 07 C 149/42, A 01 N 33/22

(21) Anmeldenummer: **83113054.7**

(22) Anmeldetag: **23.12.83**

(54) **Diphenylether, Verfahren zu ihrer Herstellung und ihre Verwendung zur Bekämpfung unerwünschten Pflanzenwuchses.**

(30) Priorität: **11.01.83 DE 3300585**

(43) Veröffentlichungstag der Anmeldung: **22.08.84 Patentblatt 84/34**

(45) Bekanntmachung des Hinweises auf die Patenterteilung: **26.02.86 Patentblatt 86/9**

(84) Benannte Vertragsstaaten: **BE CH DE FR GB IT LI**

(56) Entgegenhaltungen:
**EP-A-0 027 555**
**DE-A-2 311 638**
**US-A-4 358 308**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen (DE)**

(72) Erfinder: **Parg, Adolf, Dr., Paray-le-Monial-Strasse 8, D-6702 Bad Duerkheim (DE)**
Erfinder: **Hamprecht, Gerhard, Dr., Rote-Turm-Strasse 28, D-6940 Weinheim (DE)**
Erfinder: **Wuerzer, Bruno, Dr. Dipl.-Landwirt, Ruedigerstrasse 13, D-6701 Otterstadt (DE)**

**EP 0 116 167 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die Erfindung betrifft Diphenylether, Verfahren zu ihrer Herstellung, Herbizide, die diese Verbindungen als Wirkstoffe enthalten, sowie ihre Verwendung zur Bekämpfung unerwünschten Pflanzenwuchses.

Aus der Literatur sind herbizide Wirkstoffe aus der Klasse der Diphenylether bekannt, die in ortho-Stellung zur Nitrogruppe einen Aminorest tragen (DE-OS 23 04 006, US 4 093 446).

Es wurde gefunden, daß Diphenylether der Formel

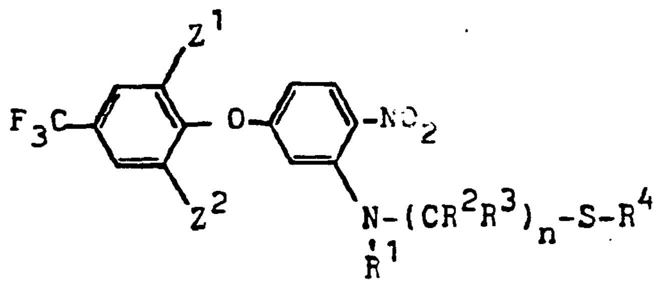

(I),

in der

$Z^1$ Wasserstoff oder Halogen,

$Z^2$ Halogen,

$R^1$, $R^2$ und $R^3$ jeweils unabhängig voneinander Wasserstoff oder $C_1$-$C_4$-Alkyl und

$R^4$ Cl-$C_{10}$-Alkyl oder einen araliphatischen Rest mit mit 7 bis 10 Kohlenstoffatomen bedeuten und

n für eine Zahl von 1 bis 8 steht,

je nach Aufbereitung und Dosierung total oder selektiv herbizid wirksam sind.

In Formel I bedeuten

$Z^1$ Wasserstoff oder Halogen, beispielsweise Fluor, Chlor oder Brom,

$Z^2$ Halogen, beispielsweise Fluor, Chlor oder Brom, $R^1$, $R^2$ und $R^3$ jeweils unabhängig voneinander Wasserstoff oder Cl-C4-Alkyl, beispielsweise Methyl, Ethyl, n-Propyl, i-Propyl, n-Butyl, i-Butyl oder tert.-Butyl,

$R^4$ $C_1$-$C_{10}$-Alkyl, beispielsweise Methyl, Ethyl, n-Propyl, i-Propyl, n-Butyl, i-Butyl, tert.-Butyl, n-Pentyl, alle isomeren Pentylreste, n-Hexyl, n-Heptyl, n-Octyl, n-Nonyl, n-Decyl und alle entsprechenden isomeren Alkylreste oder einen sraliphatischen Rest mit 7 bis 10 C-Atomen, beispielsweise Benzyl oder Phenethyl, und

n eine Zahl von 1 bis 8.

Wenn n 1 ist, können die Reste $-CR^2R^3-$ in Formel I verschieden sein.

Bevorzugte Diphenylether sind Verbindungen der Formel I, in der $Z^1$ Wasserstoff, $Z^2$ Chlor, $R^1$ Wasserstoff, $R^2$ und $R^3$ jeweils unabhängig voneinander Wasserstoff oder $C_1$-$C_4$-Alkyl und $R^4$ $C_1$-$C_{10}$-Alkyl, vorzugsweise $C_1$-$C_4$-Alkyl, bedeuten und n für eine Zahl von 1 bis 4, insbesondere 1 bis 3, steht.

Die Verbindungen der Formel I können bifspielsweise nach folgenden Verfahren hergestellt werden:

Man erhält die Diphenylether der Formel I durch Umsetzung von Diphenylethern der Formel

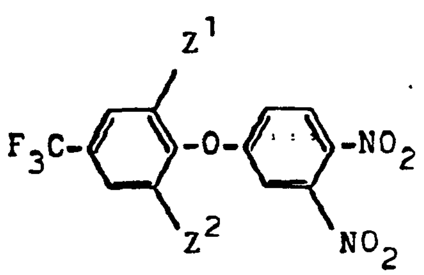

(II),

in der $Z^1$ und $Z^2$ die obengenannten Bedeutungen haben, mit der mindestens stöchiometrischen Menge eines Amins der Formel

$$H-\underset{R^1}{N}-(CR^2R^3)_n-S-R^4 \qquad (III),$$

in der $R^1$, $R^2$, $R^3$, $R^4$ und n die oben genannten Bedeutungen besitzen (Verfahren a). Die Umsetzung wird in Gegenwart eines inerten organischen Lösungsmittels bei einer Temperatur im Bereich von -20 bis +120°C, insbesondere 0 bis 100°C, kontinuierlich oder diskontinuierlich, drucklos oder unter Druck durchgeführt. Die Reaktionsdauer variiert in Abhängigkeit vom verwendeten Lösungsmittel und der Reaktionstemperatur und beträgt 0,5 bis 24 Stunden.

Außerdem erhält man die Diphenylether der Formel I durch Umsetzung von Diphenylethern der Formel

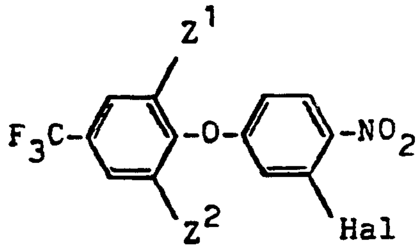

(IV),

in der $Z^1$ und $Z^2$ die obengenannten Bedeutungen haben und Hal für Halogen steht, mit der mindestens stöchiometrischen Menge eines Amins der Formel

$$H-\underset{R^1}{N}-(CR^2R^3)_n-S-R^4 \quad (III),$$

in der $R^1$, $R^2$, $R^3$, $R^4$ und n die obengenannten Bedeutungen haben, in einem inerten organischen Lösungsmittel und gegebenenfalls in Gegenwart eines Säureacceptors (Verfahren b). Die Umsetzung wird bei Temperaturen von 20 bis 180°C, insbesondere von 60 bis 150°C, kontinuierlich oder diskontinuierlich, drucklos oder unter Druck durchgeführt. Die Reaktionsdauer variiert in Abhängigkeit vom verwendeten Lösungsmittel und der Reaktionstemperatur und beträgt 2 bis 48 Stunden.

Die Herstellung der Diphenylether oder Formel I nach Verfahren a) läßt sich durch folgendes Formelschema wiedergeben:

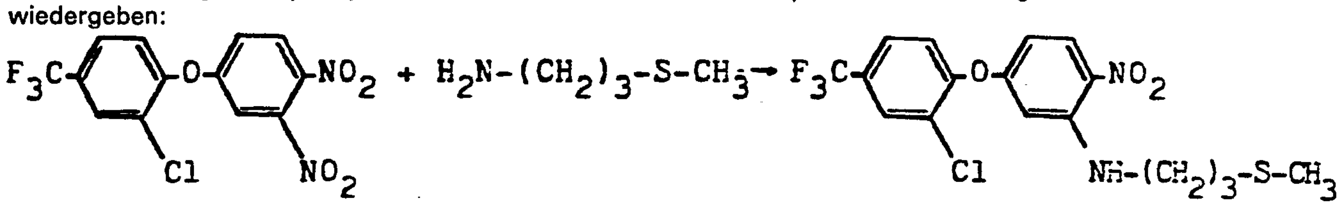

(II) (III) (I)

Die Ausgangsstoffe werden in ungefähr stöchiometrischem Verhältnis eingesetzt, vorzugsweise jedoch in einen Überschuß bis zu 200 % an Ausgangsstoff III, bezogen auf II. Zweckmäßigerweise wird das Verfahren so durchgeführt, daß man eine Lösung des Dinitrodiphenylethers II bei -20 bis +120°C, vorzugsweise bei 0 bis 100°C, in einem inerten organischen Lösungsmittel mit dem Ausgangsstoff III, gegebenenfalls gelöst in einem inerten organischen Lösungsmittel, versetzt.

Zur Beendigung der Umsetzung rührt man 0,5 bis 24 Stunden, vorzugsweise 2 bis 12 Stunden, bei 0 bis 100°C nach. Das Reaktionsgemisch wird eingeengt. Die gewünschten Endstoffe können durch Umfällen, Umkristallisieren oder durch Umrühren mit Wasser isoliert werden; gegebenenfalls können sie durch Chromatographie gereinigt werden.

Die Herstellung der Diphenylether der Formel I nach Verfahren b) läßt sich durch folgendes Formelschema wiedergeben:

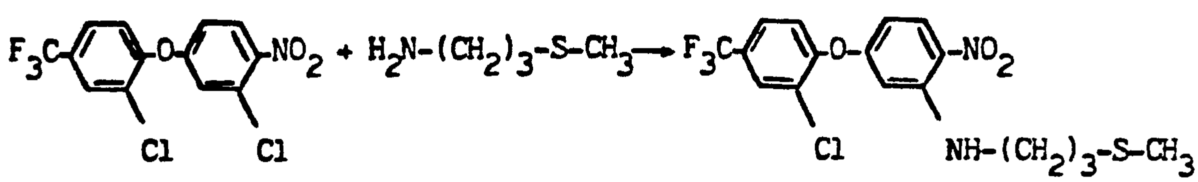

(IV) (III) (I)

Die Ausgangsstoffe werden in ungefähr stöchiometrischem Verhältnis eingesetzt d. h. in einem Unter- bzw. Überschuß von bis zu 10 % an Ausgangsstoff III bezogen auf IV. Gegebenenfalls kann ein Säureacceptor zur Vervollständigung der Reaktion zugesetzt werden, wobei diese Funktion auch von dem Ausgangsstoff III selbst übernommen werden kann. Ferner kann der bei der Umsetzung entstehende Halogenwasserstoff auch durch Einleiten eines inerten Gases, wie Stickstoff, ausgetrieben werden. Zweckmäßiger-weise wird das Verfahren so durchgeführt, daß eine Lösung des Ausgangsstoffs IV in einem inerten organischen Lösungsmittel mit der äquimolaren Menge an Ausgangsstoff III und Säureacceptor bei 20°C vermischt und zur vollständigen Umsetzung bis 180°, vorzugsweise 60 bis 150°C, erhitzt wird. Zur Beendigung der Reaktion rührt man 2 bis 48 Stunden, vorzugsweise 8 bis 24 Stunden, nach. Das Reaktionsgemisch wird eingeengt oder auf Wasser gegossen, und die gewünschten Endstoffe werden durch Extraktion oder Absaugen isoliert. Zur Reinigung kann

man umfällen, umkristallisieren oder chromatographieren.

Für beide Verfahren verwendet man unter den jeweiligen Reaktionsbedingungen inerte organische Lösungsmittel. Als Lösungsmittel kommen. z. B. in Betracht: Halogenkohlenwasserstoffe, insbesondere Chlorkohlenwasserstoffe, z. B. Tetrachlorethylen, 1,1,2,2- oder 1,1,1,2-Tetrachlorethan, Dichlorpropan, Methylenchlorid, Dichlorbutan, Chloroform, Chlornahphthalin, Dichlornaphthalin, Tetrachlorkohlenstoff, 1,1,1-oder 1,1,2-Trichlor-ethan, Trichlorethylen, Pentachlorethan, o-, m-, p-Difluorbenzol, 1,2-Dichlorethan, 1,1-Dichlorethan, 1,2-cis-Dichlorethylen, Chlorbenzol, Fluorbenzol, Brombenzol, Jodbenzol, o-, p- und m-Dichlorbenzol, o-, p-, m-Dibrombenzol, o-, m-, p-Chlortoluol, -1,2,4-Trichlorbenzol; Ether, z. B. Ethylpropylether, Methyl-tert.-butylether, n-Butylethylether, Di-n-butylether, Diisobutylether, Diisoamylether, Diisopropylether, Anisol, Phenetol, Cyclohexylmethylether, Diethylether, Ethylenglykoldimethylether, Tetrahydrofuran, Dioxan, Thioanisol, β,β'-Dichlordiethylether; Nitrokohlenwasserstoffe, wie Nitromethan, Nitroethan, Nitrobenzol, o-, m-, p-Chlornitrobenzol, o-Nitrotoluol; Nitrile, wie Acetonitril, Butyronitril, Isobutyronitril, Benzonitril, m-Chlorbenzonitril; aliphatische oder cycloaliphatische Kohlenwasserstoffe, z. B. Heptan, Pinan, Nonan, o-, m-, p-Cymol, Benzinfraktionen innerhalb eines Siedepunktintervalls von 70 bis 190°C, Cyclohexan, Methylcyclohexan, Dekalin, Petrolether, Hexan, Ligroin, 2,2,4-Triethylpentan, 2,2,3 Trimethylpentan, 2,3,3-Tri-methylpentan, Octan; Ester, z. B. Ethylacetat, Acetessigester, Isobutylacetat; Amide, z. B. Formamid, Methylformamid, Dimethylformamid; Ketone, z. B. Aceton, Methylethylketon und entsprechende andere Gemische. Zweck-mäßig verwendet man das Lösungsmittel in einer Menge von 100 bis 2 000 Cew∘-%, vorzugsweise von 200 bis 700 Gew.-%, bezogen auf die Ausgangsstoffe.

Als Säureacceptoren können alle üblichen Säurebindemittel verwendet werden. Hierzu gehören vorzugsweise tertiäre Amine, Erdalkaliverbindungen, Ammoniumverbindungen und Alkaliverbindungen sowie entsprechende Gemische. Es können aber auch Zinkverbindungen verwendet werden. Geeignet sind z.B. folgende basische Verbindungen: Kaliumhydroxid, Natriumhydroxid, Kaliumcarbonat, Natriumcarbonat, Lithiumhydroxid, Lithiumcarbonat, Natriumbicarbonat, Kaliumbicarbonat, Calciumhydroxid, Calciumoxid, Bariumoxid, Magnesiumhydroxid, Bariumhydroxid, Calciumcarbonat, Magnesiumcarbonat, Magnesiumbicarbonat, Magnesiumacetat, Zinkhydroxid, Zinkoxid, Zinkcarbonat, Zinkbicarbonat, Zinkacetat, Natriumacetat, Trimethylamin, Triethylamin, Tripropylamin, Triisopropylamin, Tributylamin, Triisobutylamin, Tri-sec.-butylamin, Tri-tert.-butylamin, Tribenzylamin, Tricyclohexylamin, Trismylamin, Diisopropylethylamin, Trihexylamin, N,N-Dimethylanilin, N,N-Diethylanilin, N,N-Dipropylanilin, N,N-Dimethyltoluoidin, N,N-Diethyltoluidin, N,N-Dipropyltoluidin, N,N-Dimethyl-p-aminopyrridin, N,N-Diethyl-p-aminopyridin, N,N-Dipropyl-p-aminopyridin, N-Methylpyrrolidon, N-Ethylpyrrolidin, N-Methylimidazol, N-Ethylimidazol, N-Methylpyrrol, N-Ethylpyrrol, N-Methylmorpholin, N-Ethylmorpholin, N-Methylhexamethylenimin, N-Ethylhexamethylenimin, Pyridin, Chinolin,α-Picolin, β-Picolin, ξPicolin, Isochinolin, Pyrimidin, Acridin, N,N,N',N'-Tetramethylethylendiamin, N,N,N',N'-Tetraethylethylendiamin, Chinoxalin, Chinazolin, N-Propyldiisopropylamin, N,N'-Dimethylcyclohexylamin, 2,6-Lutidin, 2,4-Lutidin, Trifurylamin, Triethylendiamin.

Die Ausgangsverbindungen werden nach literaturbekannten Methoden hergestellt. So erhält man 3,4-Dinitro-diphenylether der Formel II nach der in der EP-OS 7 471, 3-Chlor-4-nitro-diphenylether der Formel IV nach der in der DE-OS 2 311 638 beschriebenen Verfahrensweise. Die Verbindungen der Formel III sind allgemein zugänglich oder können ebenfalls nach literaturbekannten Verfahren hergestellt werden.

Die folgenden Beispiele erläutern die Herstellung der Verbindungen der Formel I nach den angegebenen Verfahren. Gewichtsteile verhalten sich zu Volumenteilen wie Kilogramm zu Liter.

**Beispiel 1**

Eine Lösung von 18,1 Gewichtsteilen 3,4-Dinitro-2'-chlor-4'-trifluor-methyl-diphenylether in 50 Volumenteilen Tetrahydrofuran wird mit 15 Gewichtsteilen 3-Methylthio-n-propylamin versetzt; dann wird die Reaktionsmischung 3 Stunden bei 60°C nachgerührt. Danach engt man unter vermindertem Druck zur Trockne ein, nimmt den Rückstand in Diethylether auf, extrahiert mit verdünnter Salzsäure und Wasser, trocknet die organische Phase mit Magnesiumsulfat, filtriert ab und engt erneut unter vermindertem Druck ein. Man erhält 19 Gewichtsteile (90 % d. Th.) 3-[(3''-Methyl-thio-n-propyl)-amino]-4-nitro-2'-chlor-4'-trifluormethyl-diphenylether mit dem Brechungsindex $n_D^{25}$ = 1,6079 (Verbindung Nr. 1).

**Beispiel 2**

Eine Mischung aus 21,1 Gewichtsteilen 3-Chlor-4-nitro-2'-chlor-4'-tri-fluormethyl-diphenylether, 200 Volumenteilen Pyridin, 15,3 Gewichtsteilen Natriumhydrogencarbonat und 11,3 Gewichtsteilen (1-Methyl-2-methylthio-ethyl)-amin werden 24 Stunden bei 100°C gerührt. Nach dem Abkühlen wird die Reaktionslösung in Wasser eingerührt, das abgeschiedene Oel in Methylenchlorid aufgenommen und mit verdünnter Salzsäure und Wasser extrahiert. Die organische Phase wird mit Magnesiumsulfat getrocknet, abfiltriert und unter vermindertem Druck zur Trockne eingeengt. Man erhält 22 Gewichtsteile (87 % d. Th.) 3-[(1''-Methyl-2''-methylthio-ethyl)-amino]-4 -nitro-2'-chlor-4'-trifluormethyl-diphenylether mit dem Brechungsindex $n_D^{25}$ =

1,6013 (Verbindung Nr. 2).

Entsprechend können beispielsweise die folgenden Verbindungen erhalten werden: Verbindung

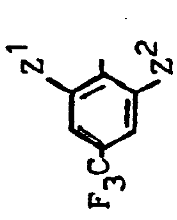

| Verbindung Nr. | | $-N(R^1)-(CR^2R^3)_n-S-R^4$ | Fp[°C]; $n_D^{25}$; Wellenlänge einer Bande im IR-Spektrum |
|---|---|---|---|
| 3 | 2-Chlor-4-trifluormethylphenyl | (2-Methylthio-ethyl)-amino | 94 - 96 |
| 4 | 2-Chlor-4-trifluormethylphenyl | (2-Ethylthio-ethyl)-amino | 1,5821 |
| 5 | 2-Chlor-4-trifluormethylphenyl | (1-Methyl-2-ethylthio-ethyl)-amino | 1,5898 |
| 6 | 2-Chlor-4-trifluormethylphenyl | (1-Methyl-2-propylthio-ethyl)-amino | 1,5848 |
| 7 | 2-Chlor-4-trifluormethylphenyl | (1,1-Dimethyl-2-methylthio-ethyl)-amino | 1,5842 |
| 8 | 2-Chlor-4-trifluormethylphenyl | (2-n-Octylthio-ethyl)-amino | 1,6092 |
| 9 | 2-Chlor-4-trifluormethylphenyl | (1-Ethyl-2-methylthio-ethyl)-amino | 1,5973 |
| 10 | 2-Chlor-4-trifluormethylphenyl | (2-Benzylthio-ethyl)-amino | 1,6181 |
| 11 | 2-Chlor-4-trifluormethylphenyl | (2-Ethylthio-n-propyl)-amino | 1,5829 |
| 12 | 2-Chlor-4-trifluormethylphenyl | (1-Ethyl-2-ethylthio-ethyl)-amino | |
| 13 | 2-Chlor-4-trifluormethylphenyl | (2-Methylthio-n-butyl)-amino | |
| 14 | 2-Chlor-4-trifluormethylphenyl | (2-Ethylthio-n-butyl)-amino | |
| 15 | 2-Chlor-4-trifluormethylphenyl | (2-n-Propylthio-propyl)-amino | |
| 16 | 2-Chlor-4-trifluormethylphenyl | (2-n-Butylthio-n-propyl)-amino | |
| 17 | 2-Chlor-4-trifluormethylphenyl | (2-Methylthio-n-butyl)-amino | |
| 18 | 2-Chlor-4-trifluormethylphenyl | (2-Ethylthio-n-butyl)-amino | |
| 19 | 2-Chlor-4-trifluormethylphenyl | (2-Phenethylthio-n-propyl)-amino | |
| 20 | 2-Chlor-4-trifluormethylphenyl | (1,1-Dimethyl-2-methylthio-n-propyl)-amino | |
| 21 | 2-Brom-4-trifluormethylphenyl | (2-Methylthio-n-propyl)-amino | |
| 22 | 2,6-Dichlor-4-trifluormethyl-phenyl | (2-Methylthio-n-propyl)-amino | |

Die Diphenylether der Forme 1 können beispielsweise in Form von direkt versprühbaren Lösungen, Pulvern Suspensionen auch hochprozentigen wäßri-gen, öligen oder sonstigen Suspensionen oder Dispersionen, Emulsionen, Öldispersionen, Pasten, Stäubemitteln, Streumitteln oder Granulaten durch Versprühen, Vernebeln,

Verstäuben, Verstreuen oder Gießen angewendet werden. Die Anwendungsformen richten sich ganz nach den Verwendungszwecken; sie sollten in jedem Fall möglichst die feinste Verteilung der erfindungs-gemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, z.B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, wie z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenoläther, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver-, Streu- und Stäubemittel können durch Mischen oder Bemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an festen Trägerstoffen hergestellt werden. Feste Trägerstoffe sind Mineralerden wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten zwischen 0,1 und 95 Gewichtsprozent, vorzugsweise zwischen 0,5 und 90 Gewichtsprozent, Wirkstoff.

Beispiele für Formulierungen sind:

I. Man vermischt 90 Gewichtsteile der Verbindung Nr. 1 mit 10 Gewichtsteilen N-Methyl-α-pyrrolidon und erhält eine Lösung, die zur Anwendung in Form kleinster Tropfen geeignet ist.

II. 10 Gewichtsteile der Verbindung Nr. 2 werden in einer Mischung gelöst, die aus 90 Gewichtsteilen Xylol, 6 Gewichtsteilen des Anlagerungsproduktes von 8 bis 10 Mol Ethylenoxid an 1 Mol Ölsäure-N-mono-ethanolamid, 2 Gewichtsteilen Calciumsalz der Dodecyl-benzolsulfonsäure und 2 Gewichtsteilen des Anlagerungsproduktes von 40 Mol Ethylenoxid an 1 Mol Ricinusöl besteht.

III. 20 Gewichtsteile der Verbindung Nr. 10 werden in einer Mischung gelöst, die aus 60 Gewichtsteilen Cyclohexanon, 30 Gewichtsteilen Isobutanol, 5 Gewichtsteilen des Anlagerungsproduktes von 7 Mol Ethylenoxid an 1 Mol Isooctylphenol und 5 Gewichtsteilen des Anlagerungsproduktes von 40 Mol Ethylenoxid an 1 Mol Ricinusöl besteht.

IV. 20 Gewichtsteile der Verbindung Nr. 4 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

V. 80 Cewichtsteile der Verbindung Nr. 6 werden mit 3 Gewichtsteilen des Natriumsalzes der Diisobutylnaphthalin-sulfonsäure, 10 Gewichtsteilen des Na-triumsalzes einer Ligninsulfonsäure aus einer Sulfit-Ablauge und 7 Gewichtsteilen pulverförmigem Kieselsäuregel gut vermischt und in einer Hammermühle vermahlen.

VI. 5 Gewichtsteile der Verbindung Nr. 3 werden mit 95 Gewichtsteilen feinteiligem Kaolin vermischt. Man erhält auf diese Weise ein Stäubemittel, das 5 Gewichtsprozent des Wirkstoffs enthält.

VII. 30 Gewichtsteile der Verbindung Nr. 11 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Teile der Verbindung Nr. 9 werden mit 2 Teilen Calciumsalz der Dodecylbenzolsulfonsäure, 8 Teilen Fettalkohol-polyglykolether, 2 Teilen Natriumsalz eines Phenol-Harnstoff-Formaldehyd-Kondensates und 68 Teilen eines paraffinischen Mineralöls innig vermischt. Man erhält eine stabile ölige Dispersion.

Die Applikation kann im Vorauflauf- oder im Nachauflaufverfahren erfolgen. Sind die Wirkstoffe bei

Nachauflaufanwendung für gewisse Kulturpflanzen weniger verträglich, können auch Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (postdirected, lay-by).

Die Aufwandmengen an Wirkstoff betragen je nach Jahreszeit, Zielpflanzen und Wachstumsstadien, Bodenart und Anwendungsverfahren 0,005 bis 3,0 kg/ha, vorzugsweise 0,01 bis 1,0 kg/ha.

Die herbizide Wirkung der Diphenylether der Formel I läßt sich durch Gewächshausversuche zeigen:

Als Kulturgefäße dienen Plastikblumentöpfe mit 300 $cm^3$ Inhalt und lehmigem Sand mit etwa 1,5 % Humus als Substrat. Die Samen der Testpflanzen werden nach Arten getrennt flach eingesät. Unmittelbar danach erfolgt bei Vorauflaufbehandlung das Aufbringen der Wirkstoffe auf die Erdoberfläche. Sie werden hierzu in Wasser als Verteilungsmittel suspendiert oder emulgiert und mittels fein verteilender Düsen gespritzt. Bei dieser Applikationsmethode beträgt die Aufwandmenge 3,0 kg Wirkstoff/ha. Nach dem Aufbringen der Mittel beregnet man die Gefäße leicht, um Keimung und Wachstum in Gang zu bringen. Danach deckt man die Gefäße mit durchsichtigen Plastikhauben ab, bis die Pflanzen angewachsen sind. Diese Abdeckung bewirkt ein gleichmäßiges Keimen der Testpflanzen, sofern dies nicht durch die Wirkstoffe beeinträchtigt wird.

Zum Zwecke der Nachauflaufbehandlung zieht man die Testpflanzen je nach Wuchsform erst bis zu einer Wuchshöhe von 3 bis 15 cm an und behandelt sie danach. Es werden entweder direkt gesäte und in den gleichen Gefäßen aufgewachsene Pflanzen verwendet, oder aber solche, die erst als Keimpflanzen getrennt angezogen und einige Tage vor der Behandlung in die Versuchsgefäße verpflanzt werden. Die Aufwandmenge beträgt 0,03 bzw. 0,06 kg Wirkstoff/ha.

Die Versuchsgefäße werden im Gewächshaus aufgestellt, wobei für wärmeliebende Arten wärmere Bereiche (20 bis 35°C) und für solche gemäßigter Klimate 10 bis 25°C bevorzugt werden. Die Versuchsperiode erstreckt sich über 2 bis 4 Wochen. Während dieser Zeit werden die Pflanzen gepflegt, und ihre Reaktion auf die einzelnen Behandlungen wird ausgewertet. Bewertet wird nach einer Skala von 0 bis 100. Dabei bedeutet 100 kein Aufgang der Pflanzen bzw. völlige Zerstörung zumindest der oberirdischen Teile.

Die in den Gewächshausversuchen verwendeten Pflanzen setzen sich aus folgenden Arten zusammen:

Amaranthus spp. (Fuchsschwanz), Chenopodium album (Weißer Gänsefuß), Euphorbia geniculata (Südamerikanische Wolfsmilch), Galium aparine (Klettenlabkraut), Hordeum vulgare (Gerste), Lamium amplexicaule (Stengelumfassende Taubnessel), Oryza sative (Reis), Sesbania exaltata (Turibaum), Sinapis alba (Weißer Senf), Solanum nigrum (Schwarzer Nachtschatten), Triticum aestivum (Weizen), Viola tricolor (gewöhnliches Stiefmütterchen).

Zum Vergleich herangezogen werden die bekannten herbiziden Wirkstoffe 3-Methylamino-4-nitro-2'-chlor-4'-trifluormethyl-diphenylether (A; DE-OS 23 04 006) und 3-[(2-Hydroxy-ethyl)-amino]-4-nitro-2'-chlor-4'-trifluormethyl-diphenylether (B; US-PS 4 093 446).

Bei der Prüfung auf herbizide Eigenschaften bei Vorauflaufanwendung zeigen beispielsweise die Verbindungen Nr. 2, 9 und 10 bei einer Aufwandmenge von 3,0 kg Wirkstoff/ha eine sehr gute Aktivität.

Bei Nachsuflaufanwendung bekämpfen beispielsweise die Verbindungen Nr. 1, 2, 3, 5, 6, 7 und 9 bei einer Aufwandmenge von 0,06 kg Wirkstoff/ha breitblättrige Unkräuter sehr gut. Die Verbindungen Nr. 1 und 9 zeigen im Vergleich zu den bekannten Verbindungen A und B eine bessere Verträglichkeit für Reis bei gleichzeitig besserer Wirkung gegen breitblättrige Pflanzen, z.B. Sesbania exaltata. Außerdem bekämpfen beispielsweise die Verbindungen Nr. 1 und 11 mit nur 0,03 kg Wirkstoff/ha unerwünschte breitblättrige Pflanzen, wobei sie selektiv gegenüber Kulturpflanzen, wie Getreide und Reis, sind. Auftretende Blattverbrennungen an den Kulturpflanzen sind tolerierbar und führen nicht zu nachhaltigen Schäden.

In Anbetracht der Vielseitigkeit der Applikationsmethoden können die Diphenylether der Formel I noch in einer weiteren Zahl von Kulturpflanzen zur Beseitigung von unerwünschtem Pflanzenwuchs, vorzugsweise breitblättriger annueller Arten, eingesetzt werden. In Betrscht kommen beispielsweise folgende Kulturen:

**Botanischer Name Deutscher Name**

Ananas comosus Ananas
Arachis hypogaea Erdnuß
Asparagus officinalis Spargel
Avena sativa Hafer
Beta vulgaris spp. altissima Zuckerrübe
Beta vulgaris spp. rapa Futterrübe
Beta vulgaris spp. esculenta Rote Rübe
Brassica napus var. napus Raps
Brassica napus var. napobrassica Kohlrübe
Camellia sinensis Teestrauch
Carthamus tinctorius Saflor - Färberdistel
Carya illinoinensis Pekannußbaum

| Botanischer Name | Deutscher Name |
|---|---|
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Cynodon dactylon | Bermudagras |
| Elaeis guineensis | Ölpalme |
| Pragaria vesca | Brdbeere |
| Glycine max | Sojabohne |
| Gossypium hirsutum (Gossypium arboreum Gossypium herbaceium (Gossypium vitifolium) | Baumwolle |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Mentha piperita | Pfefferminze |
| Musa spp. | Obst- und Mehlbanane |
| Nicotiana tabacum (N. rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Erdbohne |
| Phaseolus vulgaris | Buschbohnen |

| Botanischer Name | Deutscher Name |
|---|---|
| Pennisetum glaucum | Perl- oder Rohrkolbenhirse |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Tibes uva-crispa | Stachelbeere |
| Ricinus communis | Rizinus |
| Saccharum officinarum | Zuckerrohr |
| Secale cereale | Roggen |
| Sesamum indicum | Sesam |
| Solanum tuberosum | Kartoffel |

Sorghum bicolor (s. vulgare) Mohrenhirse
Sorghum dochna Zuckerhirse
Spinacia oleracea Spinat
Theobroma cacao Kakaobaum
Trifolium pratense Rotklee
Triticum aestivum Weizen
Vaccinium corymbosum.Kulturheidelbeere
Vaccinium vitis-idaea Preißelbeere
Vicia faba Pferdebohnen
Vigna sinensis (V. unguiculata) Kuhbohne
Vitis vinifera Weinrebe
Zea mays Mais

Zur Verbreiterung des Wirkungsspektrums und zur Erzielung synergistischer Effekte können die Diphenylether der Formel I bzw. die sie enthaltenden Mittel auch mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderi-vate, Benzothiadiazinone, 2,6-Dinitroaniline, N-Phenylcarbamate, Thiolcarbamate, Halogencarbonsäuren, Triazine, Amide, Harnstoffe, Triazinone, Uracile, Benzofuranderivate, Cyclohexan-1,3-dion-derivate und andere in Betracht.

Außerdem kann es von Nutzen sein, die neuen Verbindungen allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Bekämpfung von Ernährungs- und Spurenelementmängeln eingesetzt werden. Es können auch nichtphytotoxische Öle und Ölkonzentrate zugesetzt werden.

**Patentansprüche**

1. Diphenylether der Formel

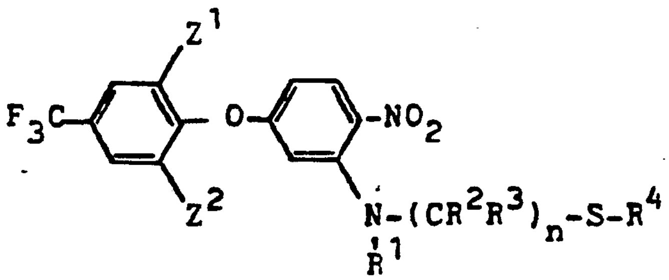

(I),

in der
$Z^1$ Wasserstoff oder Halogen,
$Z^2$ Halogen,
$R^1$, $R^2$ und $R^3$ jeweils unabhängig voneinander Wasserstoff oder $C_1$-$C_4$-Alkyl und
$R^4$ $C_1$-$C_{10}$-Alkyl oder einen araliphatischen Rest mit 7 bis 10 C-Atomen bedeuten und
n für eine Zahl von 1 bis 8 steht.

2. Diphenylether der Formel I gemäß Anspruch 1, dadurchgekennzeichnet, daß $Z^1$ Wasserstoff, $Z^2$ Chlor, $R^1$ Wasserstoff, $R^2$ und $R^3$ jeweils unabhängig voneinander Wasserstoff oder $C_1$-$C_4$-Alkyl und $R^4$ $C_1$-$C_{10}$-Alkyl bedeuten und n für eine Zahl von 1 bis 4 steht.

3. Diphenylether der Formel I gemäß Anspruch 1, dadurchgekennzeichnet, daß $Z^1$ Wasserstoff, $Z^2$ Chlor, $R^1$ Wasserstoff, $R^2$ und $R^3$ jeweils unabhängig voneinander Wasserstoff oder $C_1$-$C_4$-Alkyl und $R^4$ $C_1$-$C_4$-Alkyl bedeuten und n für eine Zahl von 1 bis 3 steht.

4. Verfahren zur Herstellung von Diphenylethern der Formel I gemäß Anspruch 1, dadurch gekennzeichnet, daß man einen 3,4-Dinitro-diphenyl-ether der Formel

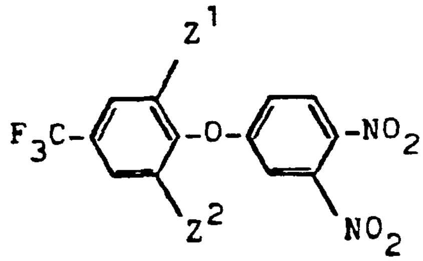

(II),

in der $Z^1$ und $Z^2$ die im Anspruch 1 genannten Bedeutungen haben, mit der mindestens stöchiometrischen Menge eines Amins der Formel

$H-N-(CR^2R^3)_n-S-R^4$ (III),
$R^1$

in der $R^1$, $R^2$, $R^3$, $R^4$ und n die im Anspruch 1 genannten Bedeutungen haben, in Gegenwart eines inerten organischen Lösungsmittels bei einer Temperatur im Bereich von -20 bis +120°C umsetzt.

5. Verfahren zur Herstellung von Diphenylethern der Formel I gemäß Anspruch 1, dadurch gekennzeichnet, daß man einen Diphenylether der Formel

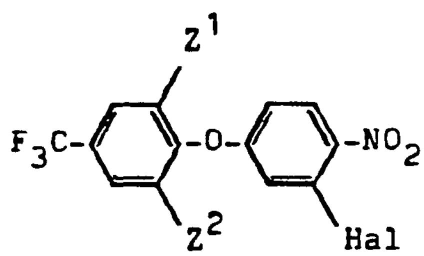

(IV),

in der $Z^1$ und $Z^2$ die in Anspruch 1 genannten Bedeutungen haben und Hal für Halogen steht, mit der mindestens stöchiometrischen Menge eines Amins der Formel

$H-N-(CR^2R^3)_n-S-R^4$ (III),
$R^1$

in der $R^1$, $R^2$, $R^3$, $R^4$ und n die im Anspruch 1 genannten Bedeutungen haben, in einem inerten organischen Lösungsmittel und gegebenenfalls in Gegenwart eines Säureacceptors bei einer Temperatur zwischen 20 und 180°C umsetzt.

6. Herbizid, enthaltend einen Diphenylether der Formel I gemäß Anspruch 1.

7. Herbizid, enthaltend inerte Zusatzstoffe und einen Diphenylether der Formel I gemäß Anspruch 1.

8. Herbizid, enthaltend inerte Zusatzstoffe und einen Diphenylether der Formel I gemäß Anspruch 2.

9. Herbizid, enthaltend inerte Zusatzstoffe und einen Diphenylether der Formel I gemäß Anspruch 3.

10. Verfahren zur Bekämpfung unerwünschten Pflanzenwachstums, dadurch gekennzeichnet, daß man die unerwünschten Pflanzen oder die von unerwünschtem Pflanzenwachstum freizuhaltende Fläche mit einer herbizid wirksamen Menge eines Diphenylethers der Formel I gemäß Anspruch 1 behandelt.

**Claims**

1. A diphenyl ether of the formula

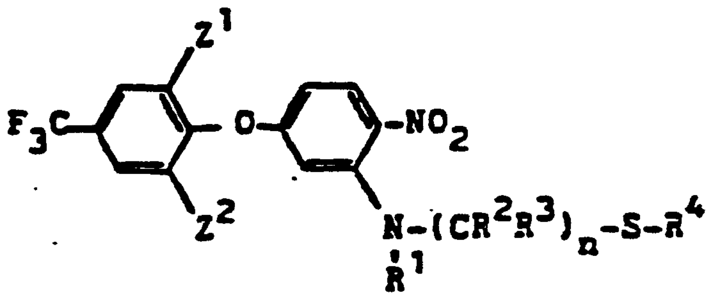

(I),

where
$Z^1$ is hydrogen or halogen,
$Z_2$ is halogen,
$R^1$, $R^2$ and $R^3$ independently of one another are hydrogen or $C_1$-$C_4$-alkyl,
$R^4$ is $C_1$-$C_{10}$-alkyl or anaraliphatic radical of 7 to 10 carbon atoms, and
n is a number from 1 to 8.

2. A diphenyl ether of the formula 1 as claimed in claim 1, where $Z^1$ is hydrogen, $Z^2$ is chlorine, $R^1$ is hydrogen, $R^2$ and $R^3$ independently of each other are hydrogen or $C_1$-$C_4$-alkyl, $R^4$ is $C_1$-$C_{10}$-alkyl, and n is a number from to 4.

3. A diphenyl ether of the formula I as claimed in claim 1, where $Z^1$ is hydrogen, $Z^2$ is chlorine, $R^1$ is hydrogen, $R^2$ and $R^3$ independently of each other are hydrogen or $C_1$-$C_4$-alkyl, $R^4$ is $C_1$-$C_{10}$-alkyl, and n is a number from to 3.

4. A process for the manufacture of a diphenyl ether of the formula I as claimed in claim 1, wherein a 3,4-dinitro-diphenyl ether of the formula

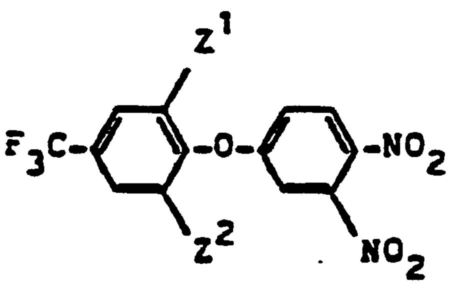

(II),

where $Z^1$ and $Z^2$ have the meanings given in claim 1, is reacted with not less than the stoichiometric amount of an amine of the formula

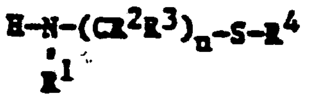

(III),

where $R^1$, $R^2$, $R^3$, $R^4$ and n have the meanings given in claim 1, in the presence of an inert organic solvent and at from -20°o +120°C.

5. A process for the manufacture of a diphenyl ether of the formula 1 as claimed in claim 1, wherein a diphenyl ether of the formula

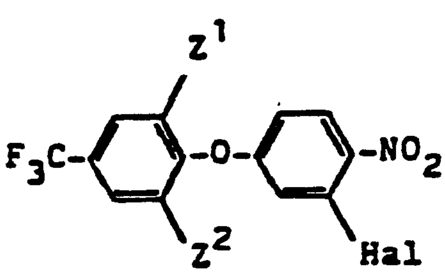

(IV),

where $Z^1$ and $Z^2$ have the meanings given in claim 1 and Hal is halogen, is reacted with not less than the stoichiometric amount of an amine of the formula

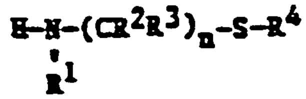

(III),

where $R^1$, $R^2$, $R^3$, $R^4$ and n have the meanings given in claim 1, in an inert organic solvent and in the presence or absence of an acid acceptor, and at from 20° to 180°C.

6. A herbicide containing a diphenyl ether of the formula I as claimed in claim 1.

7. A herbicide containing inert additives and a diphenyl ether of the formula I as claimed in claim 1.

8. A herbicide containing inert additives and a diphenyl ether of the formula I as claimed in claim 2.

9. A herbicide containing inert additives and a diphenyl ether of the formula I as claimed in claim 3

10. A process for combatting the growth of unwantedplants, wherein the unwanted plants or the area to be kept free from unwanted plant growth are treated with a herbicidally effective amount of a diphenyl ether of the formula I as claimed in claim 1.

**Revendications**

1.- Diphényléther de formule

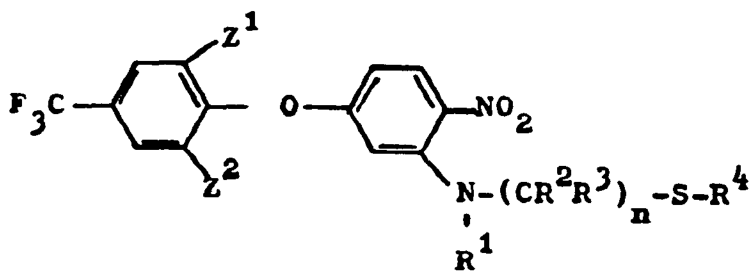

(I)

dans laquelle
$Z^1$ représente hydrogène ou halogène,
$Z^2$ halogene,
$R^1$, $R^2$ et $R^3$ respectivement, indépendants les uns des autres, hydrogène ou alkyle en $C_1$-$C_4$ et
$R^4$ représente alkyle en $C_1$-$C_{10}$ ou un reste araliphatique ayant 7 à 10 atomes C et
n est mis pour un nombre de 1 à 8.

2.- Diphenyléther de formule 1 selon la revendication 1, caractérisé par le fait que $Z^1$ représente hydrogène, $Z^2$ chlore, $R^1$ hydrogène, $R^2$ et $R^3$ respectivement indépendants les uns des autres, hydrogène ou alkyle en $C_1$-$C_4$ et $R^4$ représente alkyle en $C_1$-$C_{10}$ et n est mis pour un nombre de 1 à 4.

3.- Diphenyléther de formule 1, selon la revendication 1, caractérisé par le fait que $Z^1$ représente hydrogène, $Z^2$ chlore, $R^1$ hydrogène, R2 et $R^3$ respectivement, indépendants représente alkyle en $C_1$-$C_4$ et n est mis pour un nombre de 1 à 3.

4.- procédé de préparation de diphényléthers de formule I selon la revendication 1, caractérisé par le fait que l'on fait réagir, en présence d'un solvant organique inerte, à une température comprise entre -20 et +120°C, un 3,4-dinitro-diphényléther de formule

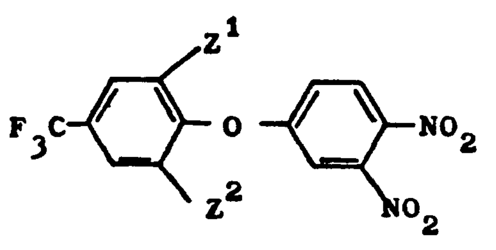

(II)

dans laquelle $Z^1$ et $Z^2$ ont les significations indiquées dans la revendication 1, avec la quantité au moins stoechiométrique d'une amine de formule

$$H-N-(CH^2R^3)_n-S-R^4 \qquad (III)$$
$$R^1$$

dans laquelle $R^1$, $R^2$, $R^3$, $R^4$ et n ont les significations indiquées dans la revendication 1.

5.- procédé de préparation de diphényléthers de formule I selon la revendication 1, caractérisé par le fait que l'on fait réagir,dans un solvant organique inerte et éventuellement en présence d'un accepteur d'acide, à une température comprise entre 20 et 180° C, un diphényléther de formule

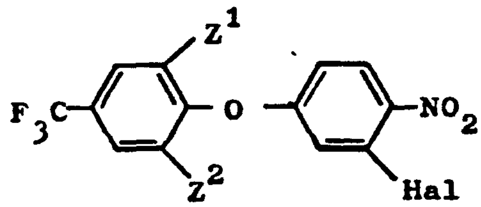

(IV)

dans laquelle $Z^1$ et $Z^2$ ont les sgnifications indiquées dans la revendication 1 et Hal est mis pour halogéne, avec la quantité au moins stoechiométrique d'une amine de formule

$$H-N-(CR^2R^3)_n-S-R^4 \qquad (III)$$
$$R^1$$

dans laquelle $R^1$, $R^2$, $R^3$, $R^4$ et n ont les significations indiquées dans la revendication 1.

6.- Herbicide contenant un diphényléther de formule I selon la revendication 1.

7.- Herbicide contenant des additifs inertes et un diphényléther de formule I selon la revendication 1.

8.- Herbicide contenant des additifs inerteset un diphényléther de formule I selon la revendication 2.

9.- Herbicide contenant des additifs inertes et un diphényléther de formule I selon la revendication 3.

10.- procédé de lutte contre la croissance indésirable de plantes, caractérisé par le fait que l'on traite les plantes indésirables, ou les surfaces à maintenir débarassées de la croissance des plantes indésirables, avec une quantité efficace du point de vue herbicide d'un diphényléther de formule I selon la revendication 1.